# EUROPÄISCHE PATENTSCHRIFT

(11) **EP 0 201 904 B1**
(45) Veröffentlichungstag und Bekanntmachung des Hinweises auf die Patenterteilung: **21.04.1993**
(21) Anmeldenummer: 86106440.0
(22) Anmeldetag: 12.05.1986
(51) Int. Cl.: C12N 15/82, C12N 1/20, A01H 1/00

(54) **Verfahren zur genetischen Modifikation von Pflanzen**
Method for the genetic modification of plants
Méthode de modification génétique de plantes

(30) Priorität: 13.05.1985 CH 2026/85
(43) Veröffentlichungstag der Anmeldung: 20.11.1986
(73) Patentinhaber: CIBA-GEIGY AG, 4002 Basel (CH)
(72) Erfinder: Grimsley, Nigel Harry, Dr., CH-4058 Basel (CH); Hohn, Barbara, Dr., CH-4103 Bottmingen (CH); Hohn, Thomas, Dr., CH-4103 Bottmingen (CH)
(74) Vertreter: Zumstein, Fritz, Dr.

(56) Entgegenhaltungen:
- EP-A- 0 116 718
- EP-A- 0 126 546
- WO-A-84/02920
- SCIENCE, Vol. 222, No. 4625, November 18, 1983 (Washington D.C.) A. CAPLAN et al. "Introduction Of Genetic Material into Plant Cells" pp. 815-821

## Beschreibung

Die vorliegende Erfindung betrifft ein neues Verfahren für die Einschleusung viraler DNS in pflanzliches Material und die nach diesem Verfahren erhältlichen pflanzlichen Produkte.

Es ist bereits in zahlreichen Fällen gelungen, ausgewählte DNS-Fragmente in Virus-DNS einzubauen und dann mit dem Virus in einen anderen Organismus einzuschleusen. Während die meisten Pflanzenviren unter natürlichen Bedingungen durch Insekten übertragen werden, welche bei der Nahrungsaufnahme an infizierten und nicht-infizierten Pflanzen fressen und hierdurch eine Neuinfektion von Pflanzen verursachen, ist dieser Weg für eine gezielte und systematische Uebertragung zu aufwendig und zu schwer kontrollierbar. So würden beispielsweise für eine solche Methode unter kontrollierten Bedingungen herangezüchtete Insektenpopulationen benötigt. Ferner wäre insbesondere bei grossen Mengen an Pflanzenmaterial eine planmässige Virusinfektion sehr schwierig zu erreichen. Die in der Gentechnologie angewendete Methode der mechanischen Inokulation von Blättern mit Viren ist nur begrenzt anwendbar, da klonierte virale DNS nur in manchen Fällen infektiös ist, in vielen anderen Fällen dagegen noch nicht. Obwohl es möglich ist, gewisse Arten von Virusgenomen in Bakterien zu klonieren und zu studieren, wie beispielsweise Einzelstrang-DNS-Viren, welche durch Klonierung von Doppelstrang-DNS erhalten werden (¹⁾Mullineaux, P.M. et al., 1984), können doch viele in Bakterien klonierte Viren nicht wieder in die Pflanzen eingeschleust oder zur Infektion von pflanzlichem Material verwendet werden. Damit ist aber auch die Anwendung von Methoden wie der in-vitro-Mutagenese und der Rekombinanten-DNS-Technologie für grundlegende Untersuchungen wie auch für den Einsatz solcher Viren als Träger von ausgewählter Fremd-DNS ausgeschlossen. Derartige Probleme treten bei Anwendung des nachstehend beschriebenen, erfindungsgemässen Verfahrens nicht auf.

Im Rahmen der vorliegenden Erfindung gelten folgende Definitionen:

| | |
|---|---|
| Transfer-Mikroorganismus: | Mikroorganismus, der einen Teil seiner DNS in pflanzliches Material überführen kann (beispielsweise Agrobacterium tumefaciens) |
| T-Replikon: | ein Replikon (²⁾Jacob, F. et al., (1963), welches mit Hilfe von Genen, die auf diesem Replikon selbst oder auf einem anderen, im selben Mikroorganismus vorhandenen Replikon lokalisiert sind, in seiner Gesamtheit oder als Teilstück in Pflanzenzellen transportiert werden kann (Beispiel: das Ti-Plasmid von Agrobacterium tumefaciens) |
| T-DNS-Grenz-Sequenzen ("border sequences"): | DNS-Sequenzen, die in einer oder mehreren Kopien mit Hilfe mikrobieller Funktionen DNS-Transfer in pflanzliches Material bewirken |
| Cargo-DNS: | in einen DNS-Vektor artifiziell eingesetzte DNS |
| Pflanzliche Zellkulturen: | Kulturen von pflanzlichen Einheiten wie beispielsweise Protoplasten, Zellkulturzellen, Zellen in Pflanzengeweben, Pollen, Pollenschläuche, Eizellen, Embryosäcke, Zygoten und Embryonen in unterschiedlichen Entwicklungsstadien |
| Vollständig durchtransformierte Pflanzen: | Pflanzen, in denen das Genomen jeder Zelle im gewünschten Sinne transformiert ist. |

Die vorliegende Erfindung betrifft ein neues Verfahren für die Einschleusung viraler DNS in pflanzliches Material, insbesondere in ganze Pflanzen oder pflanzliche Zellkulturzellen, welches generell anwendbar ist. Das Verfahren besteht im wesentlichen darin, dass man
a) in ein T-Replikon, wie beispielsweise ein Ti-Plasmid oder ein Ri-Plasmid eines Agrobacteriums, virale DNS, beispielsweise DNS von Cauliflower-Mosaik-Virus (CaMV), welche gewünschtenfalls Cargo-DNS eingebaut enthält, in Nachbarschaft zu einer oder mehreren T-DNS-Grenz-Sequenzen einsetzt, wobei die Entfernung zwischen viraler DNS und T-DNS-Grenz-Sequenz(en) so gewählt ist, dass die Uebertragung der viralen DNS einschliesslich gegebenenfalls vorhandener Cargo-DNS in pflanzliches Material erfolgt,
b) anschliessend die Aufnahme des Replikons in einen Transfer-Mikroorganismus veranlasst, wobei das Replikon in den Transfer-Mikroorganismus gelangt, und
c) mit dem gemäss b) veränderten Transfer-Mikroorganismus pflanzliches Material infiziert.

Dieses Verfahren gewährleistet, dass nach Induzierung der mikrobiellen Funktionen, welche den Transfer der Plasmid-DNS in pflanzliches Material fördern, auch die eingesetzte Virus-DNS übertragen wird, einschliesslich der gegebenenfalls vorhandenen Cargo-DNS. Das so erhaltene transformierte pflanzliche Material kann zu vollständig durchtransformierten Pflanzen regeneriert werden.

Das erfindungsgemässe Verfahren besteht somit im wesentlichen aus folgenden Teilschritten:
a) Isolierung von viraler DNS oder ihren Aequivalenten (s. weiter unten) aus infiziertem pflanzlichem Material, beispielsweise solchem der Gattung Brassica, und Klonierung dieser DNS in Vektoren eines geeigneten Bakteriums, wie beispielsweise Escherichia coli;
b) Aufbau eines Plasmids (=BaP), welches mehr als ein virales Genom oder Teile viraler Genome enthält die noch in der Lage sind eine systemische Infektion in der Wirtspflanze auszulösen, welche sich in Nachbarschaft zu einer oder mehreren T-DNS-Grenz-Sequenzen befinden, wobei die Entfernung zwischen viraler DNS und T-DNS-Grenz-Sequenz(en) so gewählt ist, dass die Uebertragung der viralen DNS einschliesslich der gegebenenfalls darin eingebauten Cargo-DNS in pflanzliches Material erfolgt;
c) Konstruktion eines Vektor-Systems durch Ueberführung des Plasmids BaP in einen Transfer-Mikroorganismus (beispielsweise Agrobacterium tumefaciens oder Agrobacterium rhizogenes); und
d) Infektion von pflanzlichem Material mit dem vorstehend unter c) aufgeführten Vektor-System.

Die Verwendung von Vektor-Systemen, wie sie vorstehend unter c) beschrieben sind, sowie neue Vektor-Systeme, wie beispielsweise Bakterien des Stammes Agrobacterium tumefaciens A136 (pTiBo542, pEAP18::Ca305) und Agrobacterium tumefaciens A136 (pTiBo542,pEAl) bilden weitere Aspekte der vorliegenden Erfindung.

Als T-Replikon kommt besonders ein bakterielles Replikon, wie ein Replikon von Agrobacterium, insbesondere von einem Ti- oder Ri-Plasmid eines Agrobacteriums, in Betracht.

Unter Mikroorganismen, welche ein T-Replikon enthalten, sind insbesondere Bakterien, vorzugsweise Bodenbakterien und von diesen vor allem solche der Gattung Agrobacterium, zu verstehen.

Unter viraler DNS und ihren Aequivalenten sind vorzugsweise folgende DNS-Typen zu verstehen:
- natürliche Virus-DNS (beispielsweise CaMV);
- doppelsträngige DNS-Formen von Einzelstrang-DNS-Viren (beispielsweise Gemini-Viren);
- cDNS-Kopien von Virus-RNS oder Viroid-RNS (beispielsweise von Tabak-Mosaik-Virus oder Cadang-Cadang-Viroid);
- jegliche lethalen oder lebensfähigen Mutanten von Viren;
- klonierte DNS unter dem Einfluss viraler Replikations- und/oder Expressionssignale;
- Teile von Virus-DNS;
- Aequivalente der vorstehend aufgeführten DNS-Typen in Tandem-Form und
- Aequivalente der vorstehend aufgeführten DNS-Typen mit eingebauter Cargo-DNS.

Als pflanzliches Material kommen sowohl ganze Pflanzen wie auch Pflanzenteile in Betracht. Als Pflanzenteile sind beispielsweise auch Protoplasten, Zellkulturzellen, Zellen in Pflanzengeweben, Pollen, Pollenschläuche, Eizellen, Embryosäcke oder Zygoten in unterschiedlichen Entwicklungsstadien zu nennen. Bevorzugt sind ganze Pflanzen und Zellkulturzellen.

Das erfindungsgemässe Verfahren eignet sich für die Virusinfektion aller Pflanzen, wobei von den systematischen Einheiten Gymnospermae und Angiospermae (einschliesslich Zierpflanzen) die letzteren hervorzuheben sind.

Unter den Angiospermae sind neben den Laubbäumen und Sträuchern von besonderem Interesse Pflanzen der Familien Solanaceae, Cruciferae, Malvaceae, Compositae, Liliaceae, Vitaceae, Chenopodiaceae, Rutaceae, Cucurbitaceae, Bromiliaceae, Rubiaceae, Theaceae, Musaceae oder Gramineae und der Ordnung Leguminosae und hier vor allem der Familie Papilionaceae. Bevorzugt sind Vertreter der Familien Solanaceae, Cruciferae und Gramineae.

Die Kulturpflanzen mit grossen Ernteerträgen wie Mais, Reis, Weizen, Gerste, Roggen, Hafer oder Hirse sind besonders erwähnenswert.

Zielkulturen sind beispielsweise solche von Pflanzen der Gattungen Solanum, Nicotiana, Gossypium(Baumwolle), Brassica (Raps), Beta, Pisum, Phaseolus, Glycine, Helianthus, Allium, Triticum (Weizen), Hordeum (Gerste), Avena (Hafer), Setaria, Sorghum (Hirse), Oryza (Reis), Zea (Mais), Cydonia, Pyrus, Malus, Rubus, Fragaria, Prunus, Arachis, Secale, Panicum, Saccharum, Coffea, Camellia, Musa, Ananas, Vitis, Citrus und Persea (Avocado).

Der Infektionsprozess erfolgt vor allem durch Zusammenführung des vorgängig beschriebenen Transfer-Mikroorganismus mit Protoplasten oder durch Verletzung von ganzen Pflanzen oder Gewebestücken und anschliessender Infizierung.

Die Verwendung des erfindungsgemässen Verfahrens unter Einsatz des vorstehend beschriebenen Vektor-Systems erbringt zahlreiche Vorteile gegenüber bisher angewendeten Methoden:
- Erzielung der Infektiosität von Viren, welche bisher artifiziell nicht zur Infektion gebracht werden konnten (beispielsweise Maize-Streak-Virus), unter Umgehung der natürlichen Vektoren wie beispielsweise Insekten;
- Möglichkeit der Manipulation von Virus-DNS in einem bakteriellen System wie beispielsweise E. coli;
- Erweiterung des Wirtsbereiches von Viren;
- Vereinfachung der Inokulation durch Vermeidung der DNS-Reinigung und drastische Verminderung der zur Inokulation erforderlichen Menge an Inokulum;
- Unter Kontrolle bakteriell kodierter Funktionen kann die T-DNS, einschliesslich der ausgewählten viralen DNS, in das Wirtsgenom eingebaut werden. Da nach einer Transformation mit Bakterien aus einer Einzelzelle eine ganze Pflanze regeneriert werden kann, kann virale DNS in das nukleare Genom sämtlicher Zellen einer Pflanze eingeführt werden. Solche integrierten Virusgenome können
   - auf sexuellem Wege auf die Nachkommenschaft übertragen werden;
   - eine Infektion mit überinfizierenden Viren verhindern; und
   - gegebenenfalls als Quelle dienen für weitere, gegebenenfalls auch ausgewählte Cargo-DNS enthaltende Viruskopien, welche sich via Transkription, Revers-Transkription, homologe Rekombination oder andere Methoden der Umlagerung genetischen Materials von der integrierten Kopie absetzen.
- Ferner kann möglicherweise eine Zweitinfektion ("Superinfection") von pflanzlichem Material, welches Teile viraler Genome in die nukleare DNS eingebaut enthält,
   a) zur Entwicklung besserer viraler Vektoren führen, da die Expression viraler Gene aus der nuklearen DNS vielleicht die Möglichkeit bietet, in dem die Zweitinfektion bewirkenden Virus virale DNS durch Fremd-DNS zu ersetzen; und
   b) zum besseren Verständnis der Wirt-Parasiten-Beziehungen und damit zur besseren Schützbarkeit der Pflanzen in erheblichem Masse beitragen.

Das erfindungsgemässe Verfahren lässt sich auch im Pflanzenschutz einsetzen, um Pflanzen durch einen Transfer-Mikroorganismus, wie vorstehend beschrieben, gegen Virusbefall zu "immunisieren", indem man die Pflanzen mit einem geschwächten, nicht pathogenen oder nur schwach pathogenen Virus transformiert.

Unter Anwendung des vorstehend beschriebenen Verfahrens lässt sich in das Virusgenom eingebaute Cargo-DNS ebenfalls in pflanzliches Material transportieren und darin ausbreiten. Die Ausbreitung der Viren und damit auch des durch sie transportierten Fremdgens in Pflanzen ist insbesondere dann ausserordentlich vorteilhaft, wenn die Pflanzen asexuell vermehrt werden sollen oder auf direktem Wege und in kürzester Zeit gegen schädigende Einflüsse geschützt werden sollen (beispielsweise durch Einbringen eines Resistenzgens in die Pflanzen).

Das erfindungsgemässe Verfahren ist insbesondere hervorragend dazu geeignet, ausgewählte Gene in pflanzliches Material, beispielsweise ausgewachsene Pflanzen, einzuschleusen und darin zu verbreiten.

Die Durchführung des DNS-Transfers in pflanzliches Material kann unter Verwendung eines der bekannten Systeme erfolgen, wie beispielsweise dem von ³⁾ An, G. et al., 1985, beschriebenen binären Vektor-System. Dieses Vektor-System lässt sich verbessern, indem man beispielsweise Sequenzen für eine homologe Rekombination der in die Pflanze zu überführenden DNS einbaut.

Am nachfolgenden Beispiel, in welches als Virus CaMV, als Klonierungsbakterium E. coli und als Vehikelbakterium Agrobacterium tumefaciens verwendet werden, werden Aufbau und Verwendung eines geeigneten Vektor-Systems näher illustriert. Das Beispiel lässt sich in analoger Weise auch mit Agrobacterium rhizogenes durchführen.

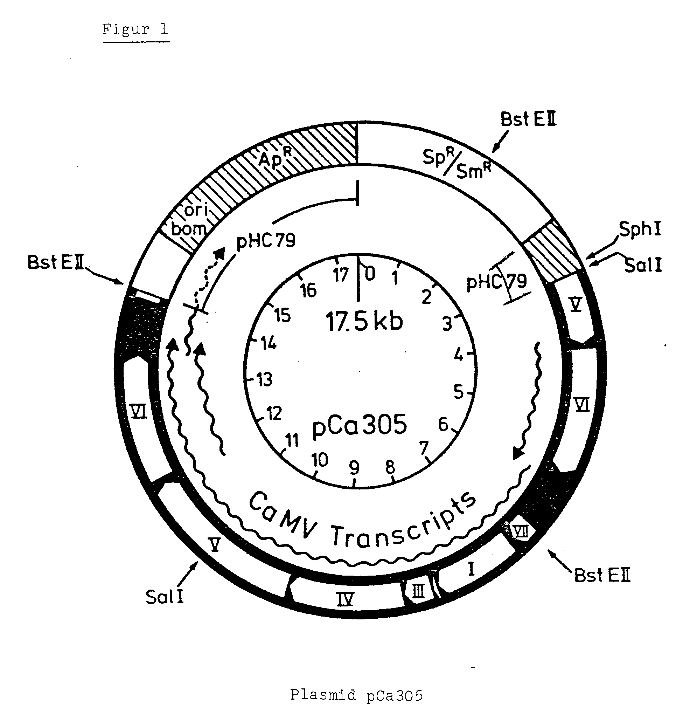

### Herstellung des bakteriellen Vektors pCa305, welcher 1,3-CaMV-Genome enthält (Fig. 1)

a) In dem Plasmid pHC79 (⁴⁾ Hohn, B. et al., 1980) wird der kleine Bereich zwischen der EcoRI-Schnittstelle und der ClaI-Schnittstelle durch das EcoRI-ClaI-Fragment, welches vom Plasmid pMON 30 (einem Vorläufer von pMON 120; ⁵⁾Fraley, R.T. et al., 1983) stammt und für Spectinomycin/Streptomycin-Resistenz kodiert, ersetzt. In dem so erhaltenen Plasmid wird der 2,5 kb umfassende Bereich zwischen den Schnittstellen SalI und BstEII durch das 3,3 kb umfassende Fragment, welches aus CaMV S (⁶⁾Hohn, T. et al., 1982) an den Schnittstellen SalI und BstEII herausgeschnitten worden ist, ersetzt. In dem so erhaltenen Plasmid pCa292 wird in die verbliebene SalI-Schnittstelle ein vollständiges CaMV CM-4184-Genom (⁷⁾Howarth, A.J. et al., 1981) eingebaut. Man erhält so das Plasmid pCa305, welches 1,3 Genome von CaMV in tandem-artiger Anordnung enthält (Fig. 1).
b) Zum Nachweis, dass die Uebertragung der infektiösen klonierten Virus-DNS auf die Empfängerpflanze nicht durch Lysierung der Agrobacterien-Zellen bewirkt wird, wird das Plasmid pGV1106 (⁸⁾Leemans, J. et al., 1982), welches einen breiten Wirtsbereich aufweist, mit dem Enzym EcoRI aufgeschnitten und die einzige SphI-Schnittstelle von pCa305 eingefügt. Das so erhaltene Plasmid pEAl wird in Agrobacterium tumefaciens eingeschleust und gelangt darin zur unabhängigen Replikation.

In Figur 1 bedeuten:
- Ap^{R}:: Ampicillin-Resistenz
- Sp^{R}/Sm^{R}:: Spectinomycin/Streptomycin-Resistenz
- ori:: Replikations-Ursprung
- bom:: Mobilisierungs-Ursprung
- BstEII, SphI, SalI:: Restriktionsstellen
- I-VII:: Offene Leseraster des Cauliflower-Mosaik-Virus
- kb:: Kilo-Basen

### Einführung der Plasmide pCa305 und pEAl in Agrobacterium tumefaciens

Die Plasmide pCa305 und pEAl werden jeweils in Bakterien des Stammes Escherichia coli GJ23 (pGJ28, R64drdll) (⁹⁾van Haute, E. et al., 1983) transformiert. Dieser E. coli-Stamm ermöglicht den konjugalen Transfer von Plasmiden, welche eine bom-Schnittstelle aufweisen, in Agrobacterium tumefaciens. Als Empfänger werden zwei verschiedene Stämme von Agrobacterium eingesetzt, welche beide von Agrobacterium tumefaciens A 136 abstammen und das Wildtyp-Ti-Plasmid pTiBo542 (¹⁰⁾ Hood, E.E. et al., 1984) enthalten.
a) Als Empfänger für das Plasmid pCa305 wird der Stamm Agrobacterium tumefaciens A136 (pTiBo542. pEAPl8) eingesetzt. Bei pEAPl8 handelt es sich um einen binären Vektor, welcher konstruiert wird, indem man das aus 6,7 kb bestehende EcoRI-BamHI-Fragment des Plasmids pGA472 (³⁾ An, G., et al., 1985) durch das aus 2,6 kb bestehende EcoRI-BglII-Fragment des Plasmids pHC79 (⁴⁾ Hohn, B. et al., 1980), welches zwischen T-DNS-Grenz-Sequenzen einen Bereich für homologe Rekombination in dem Plasmid pCa305 enthält, ersetzt. Da das Plasmid pCa305 in Agrobacterium tumefaciens nicht zur Replikation gelangt, ergibt die Selektionierung der Exkonjuganten auf Rifampicin, Spectinomycin und Streptomycin den neuen Agrobacterium-Stamm Agrobacterium tumefaciens A136 (pTiBo542, pEAPl8:: pCa305), worin das Plasmid pCa305 in den binären Vektor pEAPl8 durch homologe Rekombination integriert worden ist (¹¹⁾Leemans, J. et al., 1981);
b) Als Empfänger für das Plasmid pEAl wird der Stamm Agrobacterium tumefaciens A 136 (pTiBo542) verwendet. Die Aufnahme des Plasmids pEAl, welches in Agrobacterium tumefaciens unabhängig zur Replikation gelangt, führt zu dem neuen Stamm Agrobacterium tumefaciens A136 (pTiBo542, pEAl). Die Exkonjuganten werden auf Rifampicin (selektiv für Agrobacterium tumefaciens), Kanamycin, Spectinomycin und Streptomycin selektioniert.

Die Plasmide der Stämme, die gemäss a) und b) wie vorstehend beschrieben, erhalten werden, werden durch DNS-Isolierung und Restriktions-Kartierung geprüft.

### Uebertragung der die Plasmide pCa305 oder pEAl enthaltenden Agrobacterienstämme auf Pflanzen der Art Brassica rapa

Agrobacterien der Stämme Agrobacterium tumefaciens A136 (pTiBo542, pEAPl8::pCa305) oder Agrobacterium tumefaciens A136 (pTiBo542, pEAl) werden 40 Stunden lang in einem YEB-Medium (1 Liter Wasser enthaltend je 5 g Bacto-Rinderextrakt, Pepton und Sucrose, 1 g Bacto-Hefeextrakt, sowie 2 mM MgSO₄), welches die zur Selektionierung der Plasmide geeigneten Antibiotika Spectinomycin und/oder Streptomycin enthält, kultiviert. Anschliessend erfolgt die Anreicherung der Agrobacterien durch Zentrifugieren und Resuspendieren in 1/100 Volumen YEB (bezogen auf die vorher verwendete Menge YEB). Von dieser Suspension werden für die Inokulierung der Blätter 10 µl, für die der Blattstiele 20 µl, verwendet. Zur Inokulation werden drei Wochen alte Pflanzen von Brassica rapa c.v. Just Right verwendet, bei denen man entweder zwei Blätter pro Pflanze durch Reiben mit Celit verletzt oder nahe der Bodenoberfläche mit einem sterilen Zahnstocher ein Loch durch ein Büschel Blattstiele bohrt. Die Verletzungsstelle wird dann mit der vorstehend angegebenen Menge an Agrobacteriumsuspension beschickt.

### Ergebnisse

Während die Inokulierung mit dem Kontroll-Stamm Agrobacterium tumefaciens A136 (pTiBo542, pEAl) nicht zu einer viralen Infektion führt, wird bei dem Test-Stamm Agrobacterium tumefaciens A136 (pTiBo542, pEAPl8::pCa305) eine systemische Infektion mit CaMV erzielt, gewöhnlich in einem Zeitraum von etwa 3 Wochen. Da beide Stämme 1,3 CaMV-Genome in gleicher Anordnung enthalten, ergibt sich aus der unterschiedlichen Infektiosität, dass die Infektion der Pflanzen beim Test-Stamm eine Folge des Austretens des zwischen den T-DNS-Grenz-Sequenzen gelegenen Virus aus dem Plasmid ist, wobei die Uebertragung des Virus auf die Pflanzen durch bakteriell kodierte Funktionen gefördert wird, und dass die Infektion der Pflanzen nicht der Lysierung der Agrobacterienzellen unter Freisetzung der DNS zuzuschreiben ist.

### Literatur

1. Mullineaux, P.M., EMBO J. 3, No. 13, 3063-3068, 1984
2. Jacob, F. et al., Cold Spring Harbor Symp. 28, 329, 1963
3. An, G. et al., EMBO J. 4, No. 2, 277-284, 1985
4. Hohn, B. et al., Gene 11, 291-298, 1980
5. Fraley, R.T. et al., Proc.Natl.Acad.Sci. USA 80, 4803-4807, 1983
6. Hohn, T. et al., Current Topics of Microbiology 96, (ed. Henle et al.), 193-236, Springer-Verlag Berlin, 1982
7. Howarth, A.J. et al., Virology 112, 678-685, 1981
8. Leemans, J. et al., Gene 19, 361-364, 1982
9. Van Haute, E. et al., EMBO J. 2, No. 3, 411-417, 1983
10. Hood, E.E. et al., Bio/Technology, August 1984
11. Leemans, J. et al., Genet. 1, 149-164, 1981

## Patentansprüche (Patentansprüche für folgende(n) Vertragsstaat(en): BE, CH, DE, FR, IT, LI, LU, NL, SE)

1. Verfahren zur Einschleusung pflanzenviraler DNS in pflanzliches Material, dadurch gekennzeichnet, dass man
a) in ein T-Replikon virale DNS, welche mehr als ein virales Genom oder Teile viraler Genome umfasst die noch in der Lage sind eine systemische Injektion in der Wirtspflanze auszulösen, und welche gewünschtenfalls Cargo-DNS eingebaut einthält, in Nachbarschaft zu einer oder mehreren T-DNS-Grenz-Sequenzen einsetzt, wobei die Entfernung zwischen viraler DNS und T-DNS-Grenz-Sequenz(en) so gewählt ist, dass die Uebertragung der viralen DNS einschliesslich gegebenenfalls vorhandener Cargo-DNS in pflanzliches Material erfolgt,
b) anschliessend die Aufnahme des Replikons in einen Transfer-Mikroorganisnus veranlasst, wobei das Replikon in den Transfer-Mikroorganismus gelangt, und
c) mit dem gemäss b) veränderten Transfer-Mikroorganismus pflanzliches Material infiziert und somit eine systemische Infektion der Pflanzen verursacht.

2. Verfahren nach Anspruch 1, dadurch gekennzeichnet, dass man
a) pflanzenvirale DNS oder ihre Aequivalente aus infiziertem pflanzlichem Material isoliert und in Vektoren eines geeigneten Bakteriums kloniert;
b) ein bakterielles Plasmid (=BaP) aufbaut, welches mehr als ein pflanzenvirales Genom oder Teile pflanzenviraler Genome enthält, welche sich in Nachbarschaft zu einer oder mehreren T-DNS-Grenz-Sequenzen befinden, wobei die Entfernung zwischen viraler DNS und T-DNS-Grenz-Sequenz(en) so gewählt ist, dass die Uebertragung der pflanzenviralen DNS einschliesslich der gegebenenfalls darin eingebauten Cargo-DNS in pflanzliches Material erfolgt;
c) zur Konstruktion eines Vektor-Systems das Plasmid BaP in einen Transfer-Mikroorganismus überführt, und
d) pflanzliches Material mit dem so erhaltenen Vektor-System infiziert.

3. Verfahren nach Anspruch 1, dadurch gekennzeichnet, dass man ein bakterielles T-Replikon verwendet.

4. Verfahren nach Anspruch 1, dadurch gekennzeichnet, dass man ein T-Replikon von einem Bakterium der Gattung Agrobacterium verwendet.

5. Verfahren nach Anspruch 1, dadurch gekennzeichnet, das man als T-Replikon ein Ti-Plasmid oder ein Ri-Plasmid aus einem Bakterium der Gattung Agrobacterium verwendet.

6. Verfahren nach Anspruch 1, dadurch gekennzeichnet, dass man als Mikroorganismus, welcher das T-Replikon beherbergt, ein Bakterium verwendet.

7. Verfahren nach Anspruch 1, dadurch gekennzeichnet, dass man als Mikroorganismus, welcher das T-Replikon beherbergt, ein Bodenbakterium verwendet.

8. Verfahren nach Anspruch 1, dadurch gekennzeichnet, dass man als Mikroorganismus, welcher das T-Replikon beherbergt, ein Bakterium der Gattung Agrobacterium verwendet.

9. Verfahren nach Anspruch 1, dadurch gekennzeichnet, dass man als pflanzenvirale DNS natürliche Virus-DNS verwendet.

10. Verfahren nach Anspruch 1, dadurch gekennzeichnet, dass man als pflanzenvirale DNS solche von Cauliflower-Mosaik-Virus verwendet.

11. Verfahren nach Anspruch 1, dadurch gekennzeichnet, dass man als pflanzenvirale DNS doppelsträngige DNS-Formen von Einzelstrang-DNS-Viren verwendet.

12. Verfahren nach Anspruch 1, dadurch gekennzeichnet, dass man als pflanzenvirale DNS solche von Gemini-Viren verwendet.

13. Verfahren nach Anspruch 1, dadurch gekennzeichnet, dass man als pflanzenvirale DNS cDNS-Kopien von Virus-RNS verwendet.

14. Verfahren nach Anspruch 1, dadurch gekennzeichnet, dass man als pflanzenvirale DNS cDNS-Kopien von Viroid-RNS verwendet.

15. Verfahren nach Anspruch 1, dadurch gekennzeichnet, dass man als pflanzenvirale DNS solche von lebensfähigen Mutanten von Viren verwendet.

16. Verfahren nach Anspruch 1, dadurch gekennzeichnet, dass man als pflanzenvirale DNS Teile von Virus-DNS verwendet.

17. Verfahren nach Anspruch 1, dadurch gekennzeichnet, dass man die pflanzenvirale DNS in Tandem-Form verwendet.

18. Verfahren nach einem der Ansprüche 9 bis 16, dadurch gekennzeichnet, dass man die pflanzenvirale DNS oder Aequivalente davon in Tandem-Form verwendet.

19. Verfahren nach Anspruch 1, dadurch gekennzeichnet, das man pflanzenvirale DNS mit eingebauter Cargo-DNS verwendet.

20. Verfahren nach einem der Ansprüche 9 bis 17, dadurch gekennzeichnet, dass man pflanzenvirale DNS oder Aequivalente davon mit eingebauter Cargo-DNS verwendet.

21. Verfahren nach Anspruch 1, dadurch gekennzeichnet, dass man als pflanzliches Material ganze Pflanzen oder Pflanzenteile verwendet.

22. Verfahren nach Anspruch 1, dadurch gekennzeichnet, dass man als pflanzliches Material ganze Pflanzen oder pflanzliche Zellkulturzellen verwendet.

23. Verfahren nach Anspruch 1, dadurch gekennzeichnet, dass man als pflanzliches Material Pflanzen der Familien Solanaceae, Cruciferae, Malvaceae, Compositae, Liliaceae, Vitaceae, Chenopodiaceae, Rutaceae, Cucurbitaceae, Bromeliaceae, Rubiaceae, Theaceae, Musaceae oder Gramineae oder der Ordnung Leguminosae oder Teile dieser Pflanzen verwendet.

24. Verfahren nach Anspruch 1, dadurch gekennzeichnet, dass als pflanzliches Material Pflanzen der Familie Papilionaceae oder Teile dieser Pflanzen verwendet.

25. Verfahren nach Anspruch 1, dadurch gekennzeichnet, dass man als pflanzliches Material Pflanzen der Familien Solanaceae, Cruciferae und Gramineae oder Teile dieser Pflanzen verwendet.

26. Verfahren nach Anspruch 1, dadurch gekennzeichnet, dass man als pflanzliches Material ganze Pflanzen oder Teile von Mais, Reis, Weizen, Gerste, Roggen, Hafer oder Hirse verwendet.

27. Verfahren nach Anspruch 1, dadurch gekennzeichnet, dass man als pflanzliches Material Pflanzen der Gattungen Solanum, Nicotiana, Gossypium, Brassica, Beta, Pisum, Phaseolus, Glycine, Helianthus, Allium, Triticum, Hordeum, Avena, Setaria, Sorghum, Oryza, Zea, Cydonia, Pyrus, Malus, Rubus, Fragaria, Prunus, Arachis, Secale, Panicum, Saccharum, Coffea, Camellia, Musa, Ananas, Vitis, Persea und Citrus oder Teile dieser Pflanzen verwendet.

28. Verfahren nach Anspruch 1, dadurch gekennzeichnet, dass man die Einschleusung der viralen DNS in das pflanzliche Material unter Einsatz eines Vektor-Systems vornimmt, wobei dieses Vektor-System im wesentlichen aus einem Bakterium besteht, welches seine DNS in Pflanzenzellen transferiert und virale DNS enthält, welche in Nachbarschaft zu einer oder mehreren T-DNS-Grenz-Sequenzen vorliegt, wobei die Entfernung zwischen viraler DNS und T-DNS-Grenz-Sequenz(en) so gewählt ist, dass die Uebertragung der viralen DNS in Pflanzenzellen stattfindet, und die virale DNS gewünschtenfalls Cargo-DNS eingebaut enthält.

29. Verfahren nach Anspruch 1, dadurch gekennzeichnet, dass man Protoplasten mit dem Transfer-Mikroorganismus zusammenführt.

30. Verfahren nach Anspruch 1, dadurch gekennzeichnet, dass man ganze Pflanzen oder Gewebestücke verletzt und mit dem Transfer-Mikroorganismus infiziert.

31. Pflanzenvektor enthaltend ein bakterielles Plasmid welches mehr als ein pflanzenvirales Genom oder Teile pflanzenviraler Genome enthält, die noch in der Lage sind eine systemische Infektion in der Wirtspflanze auszulösen und welche sich in Nachbarschaft zu einer oder mehreren T-DNS-Grenz-Sequenzen befinden, wobei die Entfernung zwischen viraler DNS und T-DNS-Grenz-Sequenz(en) so gewählt ist, dass die Uebertragung der pflanzenviralen DNS einschliesslich der gegebenenfalls darin eingebauten Cargo-DNS in pflanzliches Material erfolgt.

32. Bakterien des Stammes Agrobacterium tumefaciens enthaltend einen Vektor gemäss Anspruch 31.

33. Pflanzliches Material, welches nach dem im Anspruch 1 beschriebenen Verfahren mit pflanzenviraler DNS, welche gewünschtenfalls Cargo-DNS eingebaut enthält, infiziert worden ist.

34. Vollständig durchtransformierte Pflanze, welche aus pflanzlichem Material gemäss Anspruchs 33 regeneriert worden ist.

35. Verwendung des Transfer-Mikroorganismus gemäss Anspruch 1 zur "Immunisierung" von Pflanzen gegen Virusbefall.

## Patentansprüche (Patentansprüche für folgende(n) Vertragsstaat(en): AT)

1. Verfahren zur Einschleusung pflanzenviraler DNS in pflanzliches Material, dadurch gekennzeichnet, dass man
a) in ein T-Replikon virale DNS, welche mehr als ein virales Genom oder Teile viraler Genome umfasst die noch in der Lage sind eine systemische Infektion in der Wirtspflanze auszulösen, und welche gewünschtenfalls Cargo-DNS eingebaut einthält, in Nachbarschaft zu einer oder mehreren T-DNS-Grenz-Sequenzen einsetzt, wobei die Entfernung zwischen viraler DNS und T-DNS-Grenz-Sequenz(en) so gewählt ist, dass die Uebertragung der viralen DNS einschliesslich gegebenenfalls vorhandener Cargo-DNS in pflanzliches Material erfolgt,
b) anschliessend die Aufnahme des Replikons in einen Transfer-Mikroorganismus veranlasst, wobei das Replikon in den Transfer-Mikroorganismus gelangt, und
c) mit dem gemäss b) veränderten Transfer-Mikroorganismus pflanzliches Material infiziert und somit eine systemische Infektion der Pflanzen verursacht.

2. Verfahren nach Anspruch 1, dadurch gekennzeichnet, dass man
a) pflanzenvirale DNS oder ihre Aequivalente aus infiziertem pflanzlichem Material isoliert und in Vektoren eines geeigneten Bakteriums kloniert;
b) ein bakterielles Plasmid (=BaP) aufbaut, welches mehr als ein pflanzenvirales Genom oder Teile pflanzenviraler Genome enthält, welche sich in Nachbarschaft zu einer oder mehreren T-DNS-Grenz-Sequenzen befinden, wobei die Entfernung zwischen viraler DNS und T-DNS-Grenz-Sequenz(en) so gewählt ist, dass die Uebertragung der pflanzenviralen DNS einschliesslich der gegebenenfalls darin eingebauten Cargo-DNS in pflanzliches Material erfolgt;
c) zur Konstruktion eines Vektor-Systems das Plasmid BaP in einen Transfer-Mikroorganismus überführt, und
d) pflanzliches Material mit dem so erhaltenen Vektor-System infiziert.

3. Verfahren nach Anspruch 1, dadurch gekennzeichnet, dass man ein bakterielles T-Replikon verwendet.

4. Verfahren nach Anspruch 1, dadurch gekennzeichnet, dass man ein T-Replikon von einem Bakterium der Gattung Agrobacterium verwendet.

5. Verfahren nach Anspruch 1, dadurch gekennzeichnet, das man als T-Replikon ein Ti-Plasmid oder ein Ri-Plasmid aus einem Bakterium der Gattung Agrobacterium verwendet.

6. Verfahren nach Anspruch 1, dadurch gekennzeichnet, dass man als Mikroorganismus, welcher das T-Replikon beherbergt, ein Bakterium verwendet.

7. Verfahren nach Anspruch 1, dadurch gekennzeichnet, dass man als Mikroorganismus, welcher das T-Replikon beherbergt, ein Bodenbakterium verwendet.

8. Verfahren nach Anspruch 1, dadurch gekennzeichnet, dass man als Mikroorganismus, welcher das T-Replikon beherbergt, ein Bakterium der Gattung Agrobacterium verwendet.

9. Verfahren nach Anspruch 1, dadurch gekennzeichnet, dass man als pflanzenvirale DNS natürliche Virus-DNS verwendet.

10. Verfahren nach Anspruch 1, dadurch gekennzeichnet, dass man als pflanzenvirale DNS solche von Cauliflower-Mosaik-Virus verwendet.

11. Verfahren nach Anspruch 1, dadurch gekennzeichnet, dass man als pflanzenvirale DNS doppelsträngige DNS-Formen von Einzelstrang-DNS-Viren verwendet.

12. Verfahren nach Anspruch 1, dadurch gekennzeichnet, dass man als pflanzenvirale DNS solche von Gemini-Viren verwendet.

13. Verfahren nach Anspruch 1, dadurch gekennzeichnet, dass man als pflanzenvirale DNS cDNS-Kopien von Virus-RNS verwendet.

14. Verfahren nach Anspruch 1, dadurch gekennzeichnet, dass man als pflanzenvirale DNS cDNS-Kopien von Viroid-RNS verwendet.

15. Verfahren nach Anspruch 1, dadurch gekennzeichnet, dass man als pflanzenvirale DNS solche von lebensfähigen Mutanten von Viren verwendet.

16. Verfahren nach Anspruch 1, dadurch gekennzeichnet, dass man als pflanzenvirale DNS Teile von Virus-DNS verwendet.

17. Verfahren nach Anspruch 1, dadurch gekennzeichnet, dass man die pflanzenvirale DNS in Tandem-Form verwendet.

18. Verfahren nach einem der Ansprüche 9 bis 16, dadurch gekennzeichnet, dass man die pflanzenvirale DNS oder Aequivalente davon in Tandem-Form verwendet.

19. Verfahren nach Anspruch 1, dadurch gekennzeichnet, das man pflanzenvirale DNS mit eingebauter Cargo-DNS verwendet.

20. Verfahren nach einem der Ansprüche 9 bis 17, dadurch gekennzeichnet, dass man pflanzenvirale DNS oder Aequivalente davon mit eingebauter Cargo-DNS verwendet.

21. Verfahren nach Anspruch 1, dadurch gekennzeichnet, dass man als pflanzliches Material ganze Pflanzen oder Pflanzenteile verwendet.

22. Verfahren nach Anspruch 1, dadurch gekennzeichnet, dass man als pflanzliches Material ganze Pflanzen oder pflanzliche Zellkulturzellen verwendet.

23. Verfahren nach Anspruch 1, dadurch gekennzeichnet, dass man als pflanzliches Material Pflanzen der Familien Solanaceae, Cruciferae, Malvaceae, Compositae, Liliaceae, Vitaceae, Chenopodiaceae, Rutaceae, Cucurbitaceae, Bromeliaceae, Rubiaceae, Theaceae, Musaceae oder Gramineae oder der Ordnung Leguminosae oder Teile dieser Pflanzen verwendet.

24. Verfahren nach Anspruch 1, dadurch gekennzeichnet, dass als pflanzliches Material Pflanzen der Familie Papilionaceae oder Teile dieser Pflanzen verwendet.

25. Verfahren nach Anspruch 1, dadurch gekennzeichnet, dass man als pflanzliches Material Pflanzen der Familien Solanaceae, Cruciferae und Gramineae oder Teile dieser Pflanzen verwendet.

26. Verfahren nach Anspruch 1, dadurch gekennzeichnet, dass man als pflanzliches Material ganze Pflanzen oder Teile von Mais, Reis, Weizen, Gerste, Roggen, Hafer oder Hirse verwendet.

27. Verfahren nach Anspruch 1, dadurch gekennzeichnet, dass man als pflanzliches Material Pflanzen der Gattungen Solanum, Nicotiana, Gossypium, Brassica, Beta, Pisum, Phaseolus, Glycine, Helianthus, Allium, Triticum, Hordeum, Avena, Setaria, Sorghum, Oryza, Zea, Cydonia, Pyrus, Malus, Rubus, Fragaria, Prunus, Arachis, Secale, Panicum, Saccharum, Coffea, Camellia, Musa, Ananas, Vitis, Persea und Citrus oder Teile dieser Pflanzen verwendet.

28. Verfahren nach Anspruch 1, dadurch gekennzeichnet, dass man die Einschleusung der viralen DNS in das pflanzliche Material unter Einsatz eines Vektor-Systems vornimmt, wobei dieses Vektor-System im wesentlichen aus einem Bakterium besteht, welches seine DNS in Pflanzenzellen transferiert und virale DNS enthält, welche in Nachbarschaft zu einer oder mehreren T-DNS-Grenz-Sequenzen vorliegt, wobei die Entfernung zwischen viraler DNS und T-DNS-Grenz-Sequenz(en) so gewählt ist, dass die Uebertragung der viralen DNS in Pflanzenzellen stattfindet, und die virale DNS gewünschtenfalls Cargo-DNS eingebaut enthält.

29. Verfahren nach Anspruch 1, dadurch gekennzeichnet, dass man Protoplasten mit dem Transfer-Mikroorganismus zusammenführt.

30. Verfahren nach Anspruch 1, dadurch gekennzeichnet, dass man ganze Pflanzen oder Gewebestücke verletzt und mit dem Transfer-Mikroorganismus infiziert.

31. Verfahren zur Herstellung eines Plfanzenvektors zur Übertragung pflanzenviraler DNS einschließlich der gegebenenfalls darin eingebauten Cargo-DNA in pflanzliches Material, dadurch gekennzeichnet, daß man
a) pflanzenvirale DNS oder ihre Äquivalente aus infiziertem pflanzlichem Material isoliert und in Vektoren eines geeigneten Bakteriums kloniert;
b) ein bakterielles Plasmid (=BaP) aufbaut, welches mehr als ein pflanzenvirales Genom oder Teile pflanzenviraler Genome enthält, welche sich in Nachbarschaft zu einer oder mehreren T-DNS-Grenz-Sequenzen befinden; wobei die Entfernung zwischen viraler DNS und T-DNS-Grenz-Sequenz(en) so gewählt ist, dass die Uebertragung der pflanzenviralen DNS einschliesslich der gegebenenfalls darin eingebauten Cargo-DNS in pflanzliches Material erfolgt;
c) zur Konstruktion eines Vektor-Systems das Plasmid BaP in einen Transfer-Mikroorganismus überführt.

32. Bakterien des Stammes Agrobacterium tumefaciens enthaltend einen Vektor gemäss Anspruch 31.

33. Pflanzliches Material, welches nach dem im Anspruch 1 beschriebenen Verfahren mit pflanzenviraler DNS, welche gewünschtenfalls Cargo-DNS eingebaut enthält, infiziert worden ist.

34. Vollständig durchtransformierte Pflanze, welche aus pflanzlichem Material gemäss Anspruchs 33 regeneriert worden ist.

35. Verwendung des Transfer-Mikroorganismus gemäss Anspruch 1 zur "Immunisierung" von Pflanzen gegen Virusbefall.

## Claims (Claims for the following Contracting State(s): BE, CH, DE, FR, IT, LI, LU, NL, SE)

1. A method of inserting plant-virus DNA into plant material, which comprises
a) inserting viral DNA which comprises more than one viral genome or parts thereof which are still capable of initiating a systemic infection in the host plant and which may contain cargo DNA into a T-replicon, in the vicinity of one or more T-DNA border sequences, the distance between said viral DNA and the T-DNA border sequence or sequences being chosen such that viral DNA, including any cargo DNA present, is transferred to plant material,
b) introducing the replicon into a transfer micro-organism, and
c) infecting plant material with the transfer micro-organism that has been modified in accordance with b) and thus causing a systemic infection of the plants.

2. A method according to claim 1, which comprises
a) isolating plant-virus DNA or its equivalents from infected plant material and cloning said DNA in vectors of a suitable bacterium,
b) constructing a bacterial plasmid (=BaP) that contains more than one plant-virus genome or parts thereof which are in the vicinity of one or more T-DNA border sequences, the distance between the viral DNA and the T-DNA border sequence or sequences being chosen such that said plant-virus DNA, including any cargo DNA inserted thereinto, is transferred to plant material,
c) constructing a vector system by transferring the plasmid BaP to a transfer micro-organism; and
d) infecting plant material with the vector system so obtained.

3. A method according to claim 1, wherein a bacterial T-replicon is used.

4. A method according to claim 1, wherein a T-replicon of a bacterium of the genus Agrobacterium is used.

5. A method according to claim 1, wherein the T-replicon is a Ti-plasmid or Ri-plasmid of a bacterium of the genus Agrobacterium.

6. A method according to claim 1, wherein the host micro-organism for the T-replicon is a bacterium.

7. A method according to claim 1, wherein the host micro-organism for the T-replicon is a soil bacterium.

8. A method according to claim 1, wherein the host micro-organism for the T-replicon is a bacterium of the genus Agrobacterium.

9. A method according to claim 1, wherein natural viral DNA is used as plant-virus DNA.

10. A method according to claim 1, wherein cauliflower mosaic virus is used as plant-virus DNA.

11. A method according to claim 1, wherein double-stranded DNA forms of single strand DNA viruses are used as plant-virus DNA.

12. A method according to claim 1, wherein viral DNA of gemini viruses is used as viral DNA.

13. A method according to claim 1, wherein cDNA copies of viral DNA are used as plant-virus DNA.

14. A method according to claim 1, wherein cDNA copies of viroid RNA are used as plant-virus DNA.

15. A method according to claim 1, wherein viral DNA of viable mutants of viruses is used as plant-virus DNA.

16. A method according to claim 1, wherein parts of viral DNA are used as plant-virus DNA.

17. A method according to claim 1, wherein the plant-virus DNA is used in tandem form.

18. A method according to any one of claims 9 to 16, wherein the plant-virus DNA or equivalents thereof are used in tandem form.

19. A method according to claim 1, wherein plant-virus DNA with inserted cargo DNA is used.

20. A method according to any one of claims 9 to 17, wherein plant-virus DNA or equivalents thereof with inserted cargo DNA are used.

21. A method according to claim 1, wherein the plant material comprises whole plants or parts of plants.

22. A method according to-claim 1, wherein the plant material comprises whole plants or plant cell cultures.

23. A method according to claim 1, wherein the plant material comprises plants of the families Solanaceae, Cruciferae, Malvaceae, Compositae, Liliaceae, Vitaceae, Chenopodiaceae, Rutaceae, Cucurbitaceae, Bromeliaceae, Rubiaceae, Theaceae, Musaceae or Gramineae or of the order Leguminosae, or parts thereof.

24. A method according to claim 1, wherein the plant material comprises plants of the families Papilionaceae or parts thereof.

25. A method according to claim 1, wherein the plant material comprises plants of the families Solanaceae, Cruciferae and Gramineae, or parts thereof.

26. A method according to claim 1, wherein the plant material comprises whole plants or parts of maize, rice, wheat, barley, rye, oats or sorghum.

27. A method according to claim 1, wherein the plant material comprises plants of the genera Solanum, Nicotiana, Gossypium, Brassica, Beta, Pisum, Phaseolus, Glycine, Helianthus, Allium, Triticum, Hordeum, Avena, Setaria, Sorghum, Oryza, Zea, Cydonia, Pyrus, Malus, Rubus, Fragaria, Prunus, Arachis, Secale, Panicum, Saccharum, Coffea, Camellia, Musa, Ananas, Vitis, Persea and Citrus, or parts thereof.

28. A method according to claim 1, wherein the insertion of viral DNA into the plant material is carried out using a vector system consisting substantially of a bacterium that transfers its DNA to plant cells and contains viral DNA in the vicinity of one or more T-DNA border sequences, the distance between said viral DNA and the T-DNA border sequence or sequences being chosen such that the transfer of the viral DNA to the plant cells takes place, and wherein the viral DNA may also contain inserted cargo DNA.

29. A method according to claim 1, wherein protoplasts are combined with the transfer micro-organism.

30. A method according to claim 1, which comprises wounding whole plants or pieces of tissue and infecting them with the transfer micro-organism.

31. A plant vector containing a bacterial plasmid which contains more than one plant-virus genome or parts thereof which are still capable of initiating a systemic infection in the host plant and which are in the vicinity of one or more T-DNA border sequences, the distance between said viral DNA and the T-DNA border sequence or sequences being chosen such that viral DNA, including any cargo DNA inserted thereinto, is transferred to plant material.

32. Bacterium of the strain Agrobacterium tumefaciens containing a vector according to claim 31.

33. Plant material which has been infected by the method according to claim 1 with plant-virus DNA that may contain cargo DNA inserted thereinto.

34. Completely transformed plant which has been regenerated from plant material according to claim 33.

35. Use of a transfer micro-organism according to claim 1 for "immunising" plants against virus attack.

## Claims (Claims for the following Contracting State(s): AT)

1. A method of inserting plant-virus DNA into plant material, which comprises
a) inserting viral DNA which comprises more than one viral genome or parts thereof which are still capable of initiating a systemic infection in the host plant and which may contain cargo DNA into a T-replicon, in the vicinity of one or more T-DNA border sequences, the distance between said viral DNA and the T-DNA border sequence or sequences being chosen such that viral DNA, including any cargo DNA present, is transferred to plant material,
b) introducing the replicon into a transfer micro-organism, and
c) infecting plant material with the transfer micro-organism that has been modified in accordance with b) and thus causing a systemic infection of the plants.

2. A method according to claim 1, which comprises
a) isolating plant-virus DNA or its equivalents from infected plant material and cloning said DNA in vectors of a suitable bacterium,
b) constructing a bacterial plasmid (=BaP) that contains more than one plant-virus genome or parts thereof which are in the vicinity of one or more T-DNA border sequences, the distance between the viral DNA and the T-DNA border sequence or sequences being chosen such that said plant-virus DNA, including any cargo DNA inserted thereinto, is transferred to plant material,
c) constructing a vector system by transferring the plasmid BaP to a transfer micro-organism; and
d) infecting plant material with the vector system so obtained.

3. A method according to claim 1, wherein a bacterial T-replicon is used.

4. A method according to claim 1, wherein a T-replicon of a bacterium of the genus Agrobacterium is used.

5. A method according to claim 1, wherein the T-replicon is a Ti-plasmid or Ri-plasmid of a bacterium of the genus Agrobacterium.

6. A method according to claim 1, wherein the host micro-organism for the T-replicon is a bacterium.

7. A method according to claim 1, wherein the host micro-organism for the T-replicon is a soil bacterium.

8. A method according to claim 1, wherein the host micro-organism for the T-replicon is a bacterium of the genus Agrobacterium.

9. A method according to claim 1, wherein natural viral DNA is used as plant-virus DNA.

10. A method according to claim 1, wherein cauliflower mosaic virus is used as plant-virus DNA.

11. A method according to claim 1, wherein double-stranded DNA forms of single strand DNA viruses are used as plant-virus DNA.

12. A method according to claim 1, wherein viral DNA of gemini viruses is used as plant-virus DNA.

13. A method according to claim 1, wherein cDNA copies of viral DNA are used as plant-virus DNA.

14. A method according to claim 1, wherein cDNA copies of viroid RNA are used as plant-virus DNA.

15. A method according to claim 1, wherein viral DNA of viable mutants of viruses is used as plant-virus DNA.

16. A method according to claim 1, wherein parts of viral DNA are used as plant-virus DNA.

17. A method according to claim 1, wherein the plant-virus DNA is used in tandem form.

18. A method according to any one of claims 9 to 16, wherein the plant-virus DNA or equivalents thereof are used in tandem form.

19. A method according to claim 1, wherein plant-virus DNA with inserted cargo DNA is used.

20. A method according to any one of claims 9 to 17, wherein plant-virus DNA or equivalents thereof with inserted cargo DNA are used.

21. A method according to claim 1, wherein the plant material comprises whole plants or parts of plants.

22. A method according to claim 1, wherein the plant material comprises whole plants or plant cell cultures.

23. A method according to claim 1, wherein the plant material comprises plants of the families Solanaceae, Cruciferae, Malvaceae, Compositae, Liliaceae, Vitaceae, Chenopodiaceae, Rutaceae, Cucurbitaceae, Bromeliaceae, Rubiaceae, Theaceae, Musaceae or Gramineae or of the order Leguminosae, or parts thereof.

24. A method according to claim 1, wherein the plant material comprises plants of the families Papilionaceae or parts thereof.

25. A method according to claim 1, wherein the plant material comprises plants of the families Solanaceae, Cruciferae and Gramineae, or parts thereof.

26. A method according to claim 1, wherein the plant material comprises whole plants or parts of maize, rice, wheat, barley, rye, oats or sorghum.

27. A method according to claim 1, wherein the plant material comprises plants of the genera Solanum, Nicotiana, Gossypium, Brassica, Beta, Pisum, Phaseolus, Glycine, Helianthus, Allium, Triticum, Hordeum, Avena, Setaria, Sorghum, Oryza, Zea, Cydonia, Pyrus, Malus, Rubus, Fragaria, Prunus, Arachis, Secale, Panicum, Saccharum, Coffea, Camellia, Musa, Ananas, Vitis, Persea and Citrus, or parts thereof.

28. A method according to claim 1, wherein the insertion of viral DNA into the plant material is carried out using a vector system consisting substantially of a bacterium that transfers its DNA to plant cells and contains viral DNA in the vicinity of one or more T-DNA border sequences, the distance between said viral DNA and the T-DNA border sequence or sequences being chosen such that the transfer of the viral DNA to the plant cells takes place, and wherein the viral DNA may also contain inserted cargo DNA.

29. A method according to claim 1, wherein protoplasts are combined with the transfer micro-organism.

30. A method according to claim 1, which comprises wounding whole plants or pieces of tissue and infecting them with the transfer micro-organism.

31. A method of producing a plant vector to transfer plant-virus DNA, including any cargo DNA inserted thereinto, to plant material which comprises
a) isolating plant-virus DNA or its equivalents from infected plant material and cloning said DNA in vectors of a suitable bacterium,
b) constructing a bacterial plasmid (=BaP) that contains more than one plant-virus genome or parts thereof which are in the vicinity of one or more T-DNA border sequences, the distance between the viral DNA and the T-DNA border sequence or sequences being chosen such that said plant-virus DNA, including any cargo DNA inserted thereinto, is transferred to plant material,
c) constructing a vector system by transferring the plasmid BaP to a transfer micro-organism.

32. Bacterium of the strain Agrobacterium tumefaciens containing a vector according to claim 31.

33. Plant material which has been infected by the method according to claim 1 with plant-virus DNA that may contain cargo DNA inserted thereinto.

34. Completely transformed plant which has been regenerated from plant material according to claim 33.

35. Use of a transfer micro-organism according to claim 1 for "immunising" plants against virus attack.

## Revendications (Revendications pour l'(les) Etat(s) contractant(s) suivant(s): BE, CH, DE, FR, IT, LI, LU, NL, SE)

1. Méthode pour l'introduction d'ADN viral pour les plantes dans du matériel végétal, caractérisée en ce que
a) on introduit dans un réplicon-T de l'ADN viral, qui comprend plus d'un génome viral ou des parties de génomes viraux oui sont encore en mesure de déclencher une infection systémique dans les plantes hôtes et qui éventuellement contient de l'ADN-cargo incorporé, au voisinage d'une ou plusieurs séquences limites d'ADN-T, tandis qu'on choisit l'éloignement entre ADN viraux et séquence(s) limite(s) d'ADN-T de telle sorte que le transfert de l'ADN viral, y inclus éventuellement l'ADN-cargo présent, s'effectue dans le matériel végétal,
b) on provoque ensuite l'introduction du réplicon dans un microorganisme de transfert, le réplicon pénétrant alors dans le microorganisme de transfert, et
c) on infecte du matériel végétal avec le microorganisme de transfert modifié selon b) et on provoque ainsi une infection systémique des plantes.

2. Méthode selon la revendication 1, caractérisée en ce que
a) on isole de l'ADN viral de plantes ou ses équivalents de matériel végétal infecté et on le clone dans des vecteurs d'un bacterium approprié;
b) on élabore un plasmide bactérien (=BaP) contenant plus d'un génome viral de plantes ou des parties de génomes viraux de plantes, qui se trouvent au voisinage d'une ou plusieurs séquence(s) limite(s) d'ADN-T, l'éloignement entre ADN viraux et séquence(s) limite(s) d'ADN-T étant choisi de telle sorte que le transfert de l'ADN viral de plantes, y inclus l'ADN-cargo oui y est éventuellement présent, s'effectue dans le matériel végétal;
c) pour la construction d'un système vecteur on transfère le plasmide BaP dans un microorganisme de transfert; et
d) on infecte du matériel végétal avec le système vecteur ainsi obtenu.

3. Méthode selon la revendication 1, caractérisée en ce ou'on utilise un réplicon-T bactérien.

4. Méthode selon la revendication 1, caractérisée en ce qu'on utilise un réplicon-T d'un bacterium du genre Agrobacterium.

5. Méthode selon la revendication 1, caractérisée en ce qu'on utilise comme réplicon-T un plasmide-Ti ou un plasmide-Ri d'un bacterium du genre Agrobacterium.

6. Méthode selon la revendication 1, caractérisée en ce qu'on utilise un bacterium comme microorganisme hébergeant le réplicon-T.

7. Méthode selon la revendication 1, caractérisée en ce qu'on utilise un bacterium du sol comme microorganisme hébergeant le réplicon-T.

8. Méthode selon la revendication 1, caractérisée en ce ou'on utilise un bacterium du genre Agrobacterium comme microorganisme hébergeant le réplicon-T.

9. Méthode selon la revendication 1, caractérisée en ce qu'on utilise comme ADN viral de plantes de l'ADN de virus naturel.

10. Méthode selon la revendication 1, caractérisée en ce qu'on utilise comme ADN viral de plantes de l'ADN de la mosaïque du chou-fleur.

11. Méthode selon la revendication 1, caractérisée en ce qu'on utilise comme ADN viral de plantes des formes d'ADN double brin de virus à ADN à simple brin.

12. Méthode selon la revendication 1, caractérisée en ce qu'on utilise comme ADN viral de plantes des virus Gemini.

13. Méthode selon la revendication 1, caractérisée en ce qu'on utilise comme ADN viral de plantes des copies d'ADNc d'ADN de virus.

14. Méthode selon la revendication 1, caractérisée en ce qu'on utilise comme ADN viral de plantes des copies d'ADNc d'ADN viroïde.

15. Méthode selon la revendication 1, caractérisée en ce qu'on utilise comme ADN viral de plantes des mutants stables de virus.

16. Méthode selon la revendication 1, caractérisée en ce qu'on utilise comme ADN viral de plantes des parties d'ADN de virus.

17. Méthode selon la revendication 1, caractérisée en ce qu'on utilise l'ADN viral de plantes sous une forme en tandem.

18. Méthode selon l'une quelconque des revendications 9 à 16, caractérisée en ce qu'on utilise l'ADN viral de plantes ou ses équivalents sous une forme en tandem.

19. Méthode selon la revendication 1, caractérisée en ce qu'on utilise de l'ADN viral de plantes ayant de l'ADN cargo incorporé.

20. Méthode selon l'une quelconque des revendications 9 à 17, caractérisée en ce qu'on utilise de l'ADN viral de plantes ou ses équivalents ayant de l'ADN cargo incorporé.

21. Méthode selon la revendication 1, caractérisée en ce qu'on utilise comme matériel végétal des plantes complètes ou des parties de plantes.

22. Méthode selon la revendication 1, caractérisée en ce qu'on utilise comme matériel végétal des plantes complètes ou des culturelles cellulaires de plantes.

23. Méthode selon la revendication 1, caractérisée en ce qu'on utilise comme matériel végétal des plantes des familles des: Solanaceae, Cruciferae, Malvaceae, Compositae, Liliaceae, Vitaceae, Chenopodiaceae, Rutaceae, Cucurbitaceae, Bromeliaceae, Rubiaceae, Theaceae, Musaceae ou Gramineae ou de l'ordre des Leguminosae ou des parties de ces plantes.

24. Méthode selon la revendication 1, caractérisée en ce qu'on utilise comme matériel végétal des plantes de la famille des Papilionaceae ou des parties de ces plantes.

25. Méthode selon la revendication 1, caractérisée en ce qu'on utilise comme matériel végétal des plantes des familles des Solanaceae Cruciferae et Gramineae ou des parties de ces plantes.

26. Méthode selon la revendication 1, caractérisée en ce qu'on utilise comme matériel végétal des plantes complètes de maïs, riz, blé, orge, seigle, avoine ou millet.

27. Méthode selon la revendication 1, caractérisée en ce qu'on utilise comme matériel végétal des plantes des genres Solanum, Nicotiana, Gossypium, Brassica, Beta, Pisum, Phaseolus, Glycine, Helianthus, Allium, Triticum, Hordeum, Avena, Setaria, Sorghum, Oryza, Zea, Cydonia, Pyrus, Malus, Rubus, Fragaria, Prunus, Arachis, Secale, Panicum, Saccharum, Coffea, Camellia, Musa, Ananas, Vitis, Persea et Citrus ou des parties de ces plantes.

28. Méthode selon la revendication 1, caractérisée en ce qu'on effectue l'insertion de l'ADN viral dans le matériel végétal en introduisant un système vecteur, ledit système vecteur consistant pour l'essentiel en un bacterium qui est au voisinage d'une ou plusieurs séquences limites d'ADN-T, tandis qu'on choisit l'éloignement entre ADN viraux et séquence(s) limite(s) d'ADN-T de telle sorte que le transfert de l'ADN viral s'effectue dans les cellules végétales, et l'ADN viral contient éventuellement de l'ADN cargo incorporé.

29. Méthode selon la revendication 1, caractérisée en ce qu'on réunit des protoplastes avec le microorganisme de transfert.

30. Méthode selon la revendication 1, caractérisée en ce qu'on blesse des plantes complètes ou des pièces tissulaires et on infecte avec le microorganisme de transfert.

31. Vecteur pour plantes contenant un plasmide bactérien, comprenant plus d'un génome viral de plantes ou des parties de génomes viraux de plantes qui sont encore en mesure de déclencher une infection systémique dans les plantes hôtes, qui se trouvent au voisinage d'une ou plusieurs séquences limites d'ADN-T, tandis qu'on choisit l'éloignement entre ADN viraux et séquence(s) limite(s) d'ADN-T de telle sorte que le transfert de l'ADN viral, y inclus l'ADN-cargo qui y est éventuellement incorporé, s'effectue dans le matériel végétal.

32. Bactéries du genre Agrobacterium tumefaciens contenant un vecteur selon la revendication 31.

33. Matériel végétal, qui a été infecté selon la méthode décrite dans la revendication 1 avec de l'ADN végétal, qui contient éventuellement de l'ADN cargo incorporé.

34. Plante totalement transformée, qui a été régénérée à partir de matériel végétal selon la revendication 33.

35. Utilisation du microorganisme de transfert selon la revendication 1 pour l'"immunisation" des plantes contre une attaque virale.

## Revendications (Revendications pour l'(les) Etat(s) contractant(s) suivant(s): AT)

1. Méthode pour l'introduction d'ADN viral pour les plantes dans du matériel végétal, caractérisée en ce que
a) on introduit dans un réplicon-T de l'ADN viral, qui comprend plus d'un génome viral ou des parties de génomes viraux qui sont encore en mesure de déclencher une infection systémique dans les plantes hôtes et qui éventuellement contient de l'ADN-cargo incorporé, au voisinage d'une ou plusieurs séquences limites d'ADN-T, tandis qu'on choisit l'éloignement entre ADN viraux et séquence(s) limite(s) d'ADN-T de telle sorte que le transfert de l'ADN viral, y inclus éventuellement l'ADN-cargo présent, s'effectue dans le matériel végétal,
b) on provoque ensuite l'introduction du réplicon dans un microorganisme de transfert, le réplicon pénétrant alors dans le microorganisme de transfert, et
c) on infecte du matériel végétal avec le microorganisme de transfert modifié selon b) et on provoque ainsi une infection systémique des plantes.

2. Méthode selon la revendication 1, caractérisée en ce que
a) on isole de l'ADN viral de plantes ou ses équivalents de matériel végétal infecté et on le clone dans des vecteurs d'un bacterium approprié;
b) on élabore un plasmide bactérien (=BaP) contenant plus d'un génome viral de plantes ou des parties de génomes viraux de plantes, qui se trouvent au voisinage d'une ou plusieurs séquence(s) limite(s) d'ADN-T, l'éloignement entre ADN viraux et séquence(s) limite(s) d'ADN-T étant choisi de telle sorte que le transfert de l'ADN viral de plantes, y inclus l'ADN-cargo qui y est éventuellement présent, s'effectue dans le matériel végétal;
c) pour la construction d'un système vecteur on transfère le plasmide BaP dans un microorganisme de transfert; et d) on infecte du matériel végétal avec le système vecteur ainsi obtenu.

3. Méthode selon la revendication 1, caractérisée en ce qu'on utilise un réplicon-T bactérien.

4. Méthode selon la revendication 1, caractérisée en ce qu'on utilise un réplicon-T d'un bacterium du genre Agrobacterium.

5. Méthode selon la revendication 1, caractérisée en ce qu'on utilise comme réplicon-T un plasmide-Ti ou un plasmide-Ri d'un bacterium du genre Agrobacterium.

6. Méthode selon la revendication 1, caractérisée en ce qu'on utilise un bacterium comme microorganisme hébergeant le réplicon-T.

7. Méthode selon la revendication 1, caractérisée en ce qu'on utilise un bacterium du sol comme microorganisme hébergeant le réplicon-T.

8. Méthode selon la revendication 1, caractérisée en ce qu'on utilise un bacterium du genre Agrobacterium comme microorganisme hébergeant le réplicon-T.

9. Méthode selon la revendication 1, caractérisée en ce qu'on utilise comme ADN viral de plantes de l'ADN de virus naturel.

10. Méthode selon la revendication 1, caractérisée en ce qu'on utilise comme ADN viral de plantes de l'ADN de la mosaïque du chou-fleur.

11. Méthode selon la revendication 1, caractérisée en ce qu'on utilise comme ADN viral de plantes des formes d'ADN double brin de virus à ADN à simple brin.

12. Méthode selon la revendication 1, caractérisée en ce qu'on utilise comme ADN viral de plantes des virus Gemini.

13. Méthode selon la revendication 1, caractérisée en ce qu'on utilise comme ADN viral de plantes des copies d'ADNc d'ADN de virus.

14. Méthode selon la revendication 1, caractérisée en ce qu'on utilise comme ADN viral de plantes des copies d'ADNc d'ADN viroïde.

15. Méthode selon la revendication 1, caractérisée en ce qu'on utilise comme ADN viral de plantes des mutants stables de virus.

16. Méthode selon la revendication 1, caractérisée en ce qu'on utilise comme ADN viral de plantes des parties d'ADN de virus.

17. Méthode selon la revendication 1, caractérisée en ce qu'on utilise l'ADN viral de plantes sous une forme en tandem.

18. Méthode selon l'une quelconque des revendications 9 à 16, caractérisée en ce qu'on utilise l'ADN viral de plantes ou ses équivalents sous une forme en tandem.

19. Méthode selon la revendication 1, caractérisée en ce qu'on utilise de l'ADN viral de plantes ayant de l'ADN cargo incorporé.

20. Méthode selon l'une quelconque des revendications 9 à 17, caractérisée en ce qu'on utilise de l'ADN viral de plantes ou ses équivalents ayant de l'ADN cargo incorporé.

21. Méthode selon la revendication 1, caractérisée en ce qu'on utilise comme matériel végétal des plantes complètes ou des parties de plantes.

22. Méthode selon la revendication 1, caractérisée en ce qu'on utilise comme matériel végétal des plantes complètes ou des culturelles cellulaires de plantes.

23. Méthode selon la revendication 1, caractérisée en ce qu'on utilise comme matériel végétal des plantes des familles des: Solanaceae, Cruciferae, Malvaceae, Compositae, Liliaceae, Vitaceae, Chenopodiaceae, Rutaceae, Cucurbitaceae, Bromeliaceae, Rubiaceae, Theaceae, Musaceae ou Gramineae ou de l'ordre des Leguminosae ou des parties de ces plantes.

24. Méthode selon la revendication 1, caractérisée en ce qu'on utilise comme matériel végétal des plantes de la famille des Papilionaceae ou des parties de ces plantes.

25. Méthode selon la revendication 1, caractérisée en ce qu'on utilise comme matériel végétal des plantes des familles des Solanaceae Cruciferae et Gramineae ou des parties de ces plantes.

26. Méthode selon la revendication 1, caractérisée en ce qu'on utilise comme matériel végétal des plantes complètes de maïs, riz, blé, orge, seigle, avoine ou millet.

27. Méthode selon la revendication 1, caractérisée en ce qu'on utilise comme matériel végétal des plantes des genres Solanum, Nicotiana, Gossypium, Brassica, Beta, Pisum, Phaseolus, Glycine, Helianthus, Allium, Triticum, Hordeum, Avena, Setaria, Sorghum, Oryza, Zea, Cydonia, Pyrus, Malus, Rubus, Fragaria, Prunus, Arachis, Secale, Panicum, Saccharum, Coffea, Camellia, Musa, Ananas, Vitis, Persea et Citrus ou des parties de ces plantes.

28. Méthode selon la revendication 1, caractérisée en ce qu'on effectue l'insertion de l'ADN viral dans le matériel végétal en introduisant un système vecteur, ledit système vecteur consistant pour l'essentiel en un bacterium qui est au voisinage d'une ou plusieurs séquences limites d'ADN-T, tandis qu'on choisit l'éloignement entre ADN viraux et séquence(s) limite(s) d'ADN-T de telle sorte que le transfert de l'ADN viral s'effectue dans les cellules végétales, et l'ADN viral contient éventuellement de l'ADN cargo incorporé.

29. Méthode selon la revendication 1, caractérisée en ce qu'on réunit des protoplastes avec le microorganisme de transfert.

30. Méthode selon la revendication 1, caractérisée en ce qu'on blesse des plantes complètes ou des pièces tissulaires et on infecte avec le microorganisme de transfert.

31. Méthode pour la production d'un vecteur végétal pour le transfert d'ADN viral de plantes, y compris l'ADN cargo qui s'y trouve éventuellement incorporé, dans du matériel végétal, caractérisée en ce que
a) on isole de l'ADN viral de plantes ou ses équivalents de matériel végétal infecté et on le clone dans des vecteurs d'un bacterium approprié;
b) on élabore un plasmide bactérien (=BaP) contenant plus d'un génome viral de plantes ou des parties de génomes viraux de plantes, qui se trouvent au voisinage d'une ou plusieurs séquence(s) limite(s) d'ADN-T, l'éloignement entre ADN viraux et séquence(s) limite(s) d'ADN-T étant choisi de telle sorte que le transfert de l'ADN viral de plantes, y inclus l'ADN-cargo qui y est éventuellement présent, s'effectue dans le matériel végétal;
c) pour la construction d'un système vecteur on transfère le plasmide BaP dans un microorganisme de transfert.

32. Bactéries du genre Agrobacterium tumefaciens contenant un vecteur selon la revendication 31.

33. Matériel végétal, qui a été infecté selon la méthode décrite dans la revendication 1 avec de l'ADN végétal, qui contient éventuellement de l'ADN cargo incorporé.

34. Plante totalement transformée, qui a été régénérée à partir de matériel végétal selon la revendication 33.

35. Utilisation du microorganisme de transfert selon la revendication 1 pour l'"immunisation" des plantes contre une attaque virale.
